# EUROPÄISCHE PATENTSCHRIFT

(11) **EP 4 019 076 B1**
(45) Veröffentlichungstag und Bekanntmachung des Hinweises auf die Patenterteilung: **16.11.2022**
(21) Anmeldenummer: 20217018.9
(22) Anmeldetag: 23.12.2020
(51) Int. Cl.: A61N 1/04, A61B 5/06, A61N 1/40

(54) **ELEKTROTHERAPIEGERÄT ZUR ERMITTLUNG VON POSITIONEN DES ELEKTROTHERAPIEGERÄTES**
ELECTROTHERAPY DEVICE FOR DETERMINING POSITIONS OF THE ELECTROTHERAPY DEVICE
APPAREIL D'ÉLECTROTHÉRAPIE DE DÉTERMINATION DES POSITIONS DE L'APPAREIL D'ÉLECTROTHÉRAPIE

(43) Veröffentlichungstag der Anmeldung: 29.06.2022
(73) Patentinhaber: Keytec GmbH, 86420 Diedorf (DE)
(72) Erfinder: BURR, Ulrich, 50374 Erftstadt (DE)
(74) Vertreter: Dantz, Jan Henning

(56) Entgegenhaltungen:
- EP-A1- 3 195 898
- WO-A1-2015/179744
- WO-A1-2017/035502
- RU-A- 2004 136 418
- RU-C2- 2 285 550
- US-A1- 2019 159 696

## Beschreibung

Die Erfindung betrifft ein Elektrotherapiegerät gemäß dem Oberbegriff des geltenden Anspruchs 1.

Ein Elektrotherapiegerät gemäß dem Oberbegriff des geltenden Anspruchs 1 ist aus der RU 2004 136 418 A bekannt.

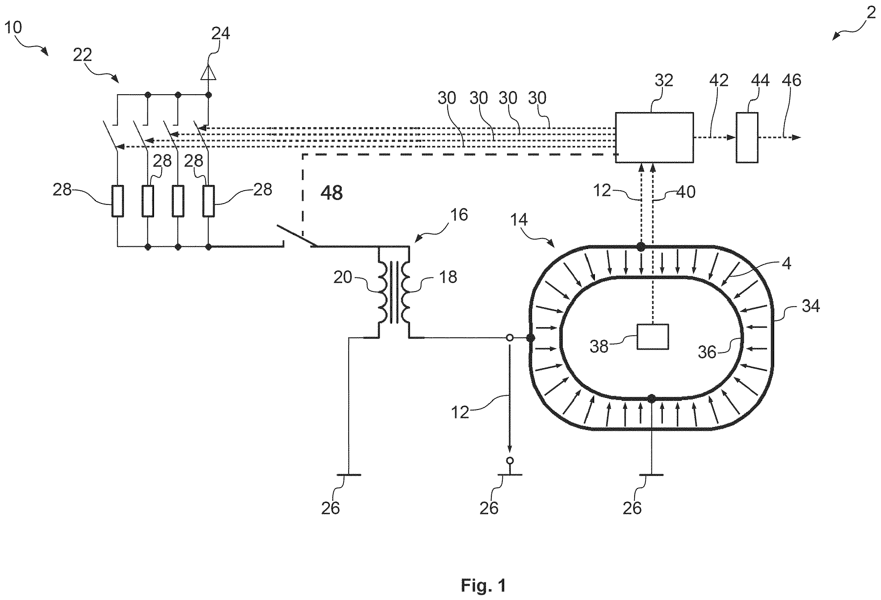

Die EP 3 195 898 A1 offenbart ein Gerät zur Stimulation der Vaginalmuskulator. Dabei umfasst eine schematisch in Fig. 1 gezeigten Ausführungsform ein Handstück mit einer einzelnen Elektrode 102 und als ein monopolares Emissionshandstück. In dieser Vorrichtung fließt der von einer HF-Stromerzeugungsmaschine außerhalb des Handstücks selbst erzeugte Strom durch die Elektrode, und der Stromkreis wird durch eine nicht isolierte Gegenelektrode geschlossen, die eine zu positionierende leitfähige Gummi- oder Metallplatte ist am Körper der von der Behandlung betroffenen Person, vorzugsweise unter dem Gluteus, oder einem in der Hand zu haltenden Zylinder. Der kapazitive Modus ermöglicht es, Gewebe mit geringem Stromwiderstand, wie z. B. Bindegewebe, gezielt zu erwärmen.

Es ist Aufgabe der Erfindung, das bekannte Elektrotherapiegerät zu verbessern.

Die vorliegende Erfindung löst die Aufgabe durch das Bereitstellen eines Elektrotherapiegerätes mit den Merkmalen des Anspruchs 1.

Gemäß einem Aspekt der Erfindung umfasst ein Elektrotherapiegerät zum Eintragen eines elektrischen Therapieimpulses in die Haut eines Patienten eine Pulsquelle zur Ausgabe einer Pulsspannung; einen auf die Haut des Patienten auflegbaren Ausgabekondensator, der mit einer ersten an einen ersten Pol der Pulsquelle angeschlossen Kondensatorelektrode und mit einer zweiten an einen zweiten Pol der Pulsquelle angeschlossen Kondensatorelektrode eingerichtet ist, die Pulsspannung in den Therapiepuls zu wandeln.

Erfindungsgemäß umfasst das Elektrotherapiegerät einen Positionssensor zum Erfassen einer Position des Ausgabekondensators auf der Haut während der Abgabe des Therapiepulses.

Der Erfindung liegt die Überlegung zugrunde, dass sich mit herkömmlichen Elektrotherapiegeräten nicht nur die Haut therapieren lässt, die Entwicklung der Therapie lässt sich unmittelbar aus dem Elektrotherapiegerät beispielsweise anhand der elektrischen Eigenschaften der Haut auslesen. So ließen sich grundsätzlich Therapieprotokolle erstellen, um die Therapie des Patienten verlässlich zu dokumentieren, sogar auf ihren Erfolg hin zu überwachen oder im Zweifel selbst mit anderen Therapieverfahren zu vergleichen. Die Verlässlichkeit und die Aussagekraft der Therapieprotokolle hängt allerdings im Wesentlichen davon ab, wie zuverlässig der Therapeut eine bestimmte Region auf der Haut mit dem Ausgabekondensator reproduzierbar ansteuert, was grundsätzlich noch durch grafische Hilfslinien auf der zu therapierenden Haut lösbar wäre. Allerdings hat der Therapeut in der Regel eine Vielzahl von anzufahrenden Therapiepunkten auf der Haut, die zuverlässig auseinandergehalten werden müssen, wenn die Therapieprotokolle ihre Aussagekraft nicht verlieren sollen.

Hier greift die Erfindung mit der Überlegung an, die Positionserfassung mit in das Elektrotherapiegerät mit aufzunehmen. Der Therapeut braucht sich auf diese Weise nicht mehr darauf zu konzentrieren, in welcher Region auf der Haut er das angegebene Elektrotherapiegerät anwendet. Die Positionsdaten können durch den Positionssensor zuverlässig und fehlerfrei erhoben werden, und steigern so die Aussagekraft der Therapieprotokolle deutlich. Der Therapeut kann sich so vielmehr auf die eigentliche Therapie konzentrieren, was potentielle Fehlerquellen ausschaltet und eine Arbeitserleichterung darstellt.

Schließlich lassen sich die Therapieprotokolle nicht nur über die Zeit, sondern auch in örtlicher Abhängigkeit auf Therapiebahnen erstellen, was die Aussagekraft der erhobenen Therapieprotokolle deutlich steigert.

In einer Weiterbildung des angegebenen Elektrotherapiegerätes umfasst die Pulsquelle einen Schwingkreis mit dem Ausgabekondensator und einer magnetisch ladbaren Spule. Eine Resonanzfrequenz des Schwingkreises, ob nun aus einer Parallelschaltung oder einer Serienschaltung des Ausgabekondensators und der Spule lässt unmittelbar Rückschlüsse auf die behandelnde Haut zu, weil die Haut die Kapazität des Ausgabekondensators verändert. Zusammen mit der erfassten Position lassen sich daher sehr präzise Therapieprotokolle erstellen, die klare Auskunft über den Behandlungsverlauf geben.

Um die Spule in einfacher Weise magnetisch zu laden, kann sie als Sekundärspule vorzugsweise Teil eines Transformators sein, die von einer Primärspule aufgeladen wird. Die verwendete Struktur des Transformators erlaubt vorzugsweise keine galvanische Trennung zwischen Primär- und Sekundärspule, da beide Wicklungen am oberen Ende verbunden sind. Dies ist vorteilhaft und Bestandteil des Konzeptes einer Bio-Feed-Back Funktion, in welcher ein Signal zur Bewertung der Therapie generiert wird. Die Haut bzw. das Gewebe liegt insbesondere immer parallel zum Sekundärkreis, beeinflusst mit seinen Eigenschaften sowohl den Vorimpuls (Aufladung) als auch den Therapieimpuls (Entladung).

Das Aufladen bzw. die Aufladung im Rahmen der vorliegenden Erfindung kann man insbesondere eine Umwandlung in magnetische Energie durch die Primärspule verstehen, die im magnetischen Feld und Transformatorkern (ferromagnetisches Material) gespeichert wird. Mit dem Abschalten der Bestromung der Primärspule wird die gespeicherte magnetische Energie wieder in elektrische Energie umgewandelt und über die Sekundärspule über die Haut abgegeben.

In einer besonderen Weiterbildung umfasst das angegebene Elektrotherapiegerät eine Sendeschnittstelle zur Übertragung der Position des Ausgabekondensators an eine Datenverarbeitungseinrichtung. Diese Sendeschnittstelle kann grundsätzlich beliebig, also kabelgebunden oder kabellos ausgeführt sein. Für eine bandbreite- und energiesparende Anbindung des angegebenen Elektrotherapiegerätes an die Datenverarbeitungseinrichtung eignet sich die Übertragung per Bluetooth Low Energie besonders, weil sich auf diese Weise auch noch andere Geräte platz-, energie- und bandbreitesparend an die Datenverarbeitungseinrichtung anschließen lassen.

In einer besonders bevorzugten Weiterbildung des angegebenen Elektrotherapiegerätes ist die Sendeschnittstelle eingerichtet, die Position des Ausgabekondensators gemeinsam mit einem die Haut an der erfassten Position beschreibenden Messwert zu versenden. Dieser Messwert kann beispielsweise die zuvor genannte Resonanzfrequenz des Schwingkreises, aber auch die Kapazität der Haut an der erfassten Position sein. Auf diese Weise lässt sich die Eigenschaft der Haut präzise örtlich auflösen, was den Therapeuten eine deutlich verbesserte Hilfe bei der Bewertung des Behandlungserfolges ist.

In einer anderen Weiterbildung des angegebenen Elektrotherapiegerätes ist der Positionssensor eingerichtet, die Position des Kondensators auf der Haut inkrementell zu erfassen. Auch wenn sich die Erfassung der Position grundsätzlich absolut beispielsweise über ein räumliches Positionssystem umsetzen lässt, erlaubt es die inkrementelle Erfassung dem Therapeuten sich selbst seine Bezugspunkte zu definieren, ausgehend von denen er die Hautmessung örtlich auflösen möchte. Hierdurch lässt sich das angegebene Elektrotherapiegerät intuitiver anwenden.

Grundsätzlich kann die inkrementelle Positionserfassung in beliebiger Weise über Kugeln, Beschleunigungssensoren, berührungslosen Abstandssensoren oder dergleichen erfasst werden. In einer zusätzlichen Weiterbildung des angegebenen Elektrotherapiegerätes ist der Positionssensor zur inkrementellen Positionserfassung eingerichtet, in regelmäßigen Abständen ein Bild der Haut zu erfassen und mit einem zuvor aufgenommenen Bild der Haut zu vergleichen. Dieses Prinzip wird bereits erfolgreich bei Computermäusen zur Steuerung eines Cursors auf einem Bildschirm verwendet. Im Gegensatz zu den anderen genannten inkrementellen Positionserfassungssystemen lässt sich die Bewegung des Ausgabekondensators auf der Haut trotz naturbedingter sphärischer Unebenheiten auf der Oberfläche des Patienten im Raum erfassen. Eine räumliche Bewegung des Kondensators auf der Haut des Patienten aufgrund der sphärischen Unebenheiten wird unmittelbar als zweidimensionale Bewegung erfasst und macht jegliche Koordinatentransformation oder dergleichen überflüssig.

In einer noch anderen Weiterbildung des angegebenen Elektrotherapiegerätes sind die Kondensatorelektroden als konzentrisch zueinander angeordnete geschlossene Schleifen ausgebildet. Auf diese Weise lässt sich auf kleinstem Raum ein vergleichsweise großer Luftspalt zwischen den beiden Kondensatorelektroden schaffen, wodurch eine hohe sensorische Sensibilität für das angegebene Elektrotherapiegerät erreicht wird.

In einer besonders zweckmäßigen Weiterbildung des angegebenen Elektrotherapiegerätes ist der Positionssensor innerhalb der inneren Schleife der konzentrisch zueinander angeordneten Schleifen, vorzugsweise in deren Zentrum angeordnet. Da das elektrische Feld zwischen den beiden Schleifen im Luftspalt aufgebaut wird, ist das innere der inneren Schleife praktisch feldfrei, analog zu einem Koaxialkabel. Auf diese Weise wird das Risiko für elektromagnetische Störungen reduziert oder ganz vermieden.

In einer noch anderen Weiterbildung des angegebenen Elektrotherapiegerätes ist der Abgabekondensator an einem Behandlungskopf mit einer den Positionssensor aufnehmenden Aussparung gehalten. Auf diese Weise lässt sich der Abgabekondensator flächig auf die zu behandelnde Haut drücken, ohne dass der Positionssensor während der Behandlung stört.

In einer weiteren Weiterbildung des angegebenen Elektrotherapiegerätes ist die Aussparung mit einer Abdeckung verschlossen ist. Auf diese Weise lässt sich der Positionssensor vor Verunreinigungen, wie beispielsweise Hautschweiß oder dergleichen schützen.

Weiterhin kann das Elektrotherapiegerät einen Beschleunigungssensor aufweisen zur Ermittlung der Beschleunigungen in zumindest zwei Achsen, insbesondere in alle drei Achsen, für eine Validierung des Signals des Positionssensors.

Das Elektrotherapiegerät kann bevorzugt als Handgerät, ein sogenanntes "handheld" ausgebildet sein. Weiterhin kann das Elektrotherapiegerät, insbesondere in der Ausgestaltung als Handgerät, batteriebetrieben und/oder akkubetrieben ausgebildet sein.

Insbesondere wenn das oben genannte Prinzip der Computermaus für den Positionssensor zum Einsatz kommt, sollte die Abdeckung transparent ausgeführt sein.

Weiterhin offenbart wird ein Verfahren zur Ermittlung von Positionen des erfindungsgemäßen Elektrotherapiegerätes auf der Haut, wobei eine Bewegung des Elektrotherapiegerätes gegenüber eines vorgegebenen Ausgangspunktes erfasst wird, so dass die Positionen als Relativpositionen gegenüber dem Ausgangsposition durch den Positionssensor erfasst wird, wobei die Positionen protokolliert werden. Das Elektrotherapiegerät kann dabei auch ohne Aussenden eines Therapieimpulses zur Abstandsmessung einzelner Hautpunkte genutzt werden.

Die oben beschriebenen Eigenschaften, Merkmale und Vorteile dieser Erfindung sowie die Art und Weise wie diese erreicht werden, werden verständlicher im Zusammenhang mit der folgenden Beschreibung der Ausführungsbeispiele, die im Zusammenhang mit der Zeichnung näher erläutert werden. Es zeigen:
Fig. 1 eine schematische Darstellung eines Elektrotherapiegerätes,
Fig. 2 eine schematische Darstellung eines Behandlungskopfes des Elektrotherapiegerätes der Fig. 1,
Fig. 3a und 3b eine schematische Darstellung einer Protokollierung einer Therapie mit dem Elektrotherapiegerät der Fig. 1, und
Fig. 4a und 4b eine schematische Darstellung einer Protokollierung einer alternativen Therapie mit dem Elektrotherapiegerät der Fig. 1.

In den Figuren werden gleiche technische Elemente mit gleichen Bezugszeichen versehen und nur einmal beschrieben. Die Figuren sind rein schematisch und geben vor allem nicht die tatsächlichen geometrischen Verhältnisse wieder.

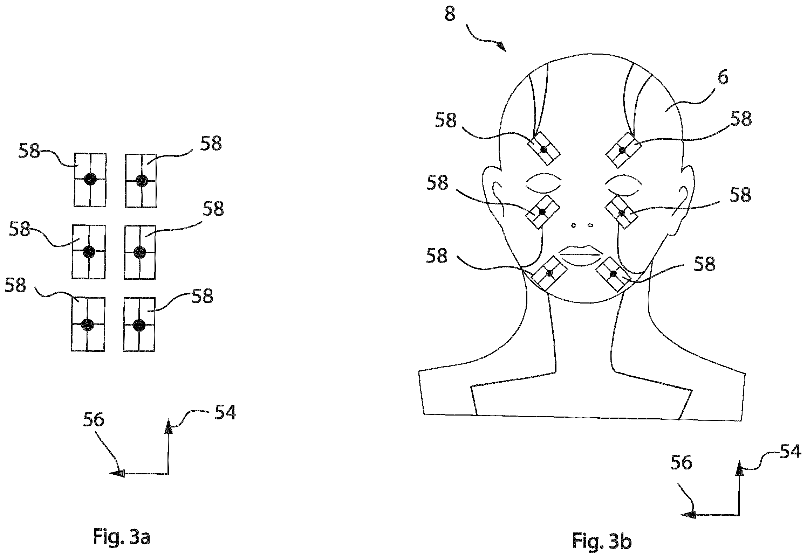

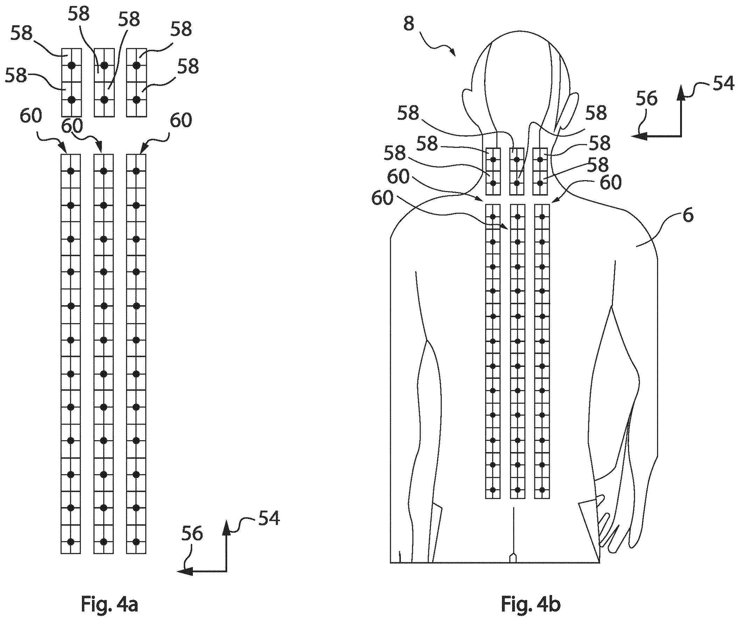

Es wird auf Fig. 1 Bezug genommen, die eine schematische Darstellung eines Elektrotherapiegerätes 2 zum Eintragen eines elektrischen Therapieimpulses 4 in die Haut 6 eines in den Fig. 3b und 4b gezeigten Patienten 8 zeigt. Der Therapiepuls 4 wird in Form eines elektrischen Feldes abgegeben, welches in Fig. 1 durch seine Feldlinien angedeutet ist.

Zur Erzeugung der Therapiepulses 4 in Form des elektrischen Feldes umfasst das Elektrotherapiegerät 2 eine Pulsquelle 10 zur Ausgabe einer Pulsspannung 12. In der vorliegenden Ausführung umfasst die Pulsquelle 10 aus einem Schwingkreis aus einem den Therapiepuls 4 ausgebenden Ausgabekondensator 14 und einem Transformator 16, der aus einer an den Ausgabekondensator 14 angeschlossenen Sekundärspule 18 und einer die Sekundärspule 18 speisenden Primärspule 20 gebildet ist.

Die Primärspule 20 ist über einen variablen Vorwiderstand 22 zwischen einem Versorgungspotential 24 und Masse 26 geschaltet. Der Vorwiderstand ist aus einer Parallelschaltung von Einzelvorwiderständen 28 gebildet, die sich über Steuersignale 30 aus einer Steuervorrichtung 32 zur Leistungsanpassung dediziert zuschalten lassen. Auf diese Weise lässt sich die Primärspule 20 mit verschiedenen Strömen bestromen, so dass sich in der Sekundärspule 18 verschiedene Magnetfelder aufbauen lassen. Wird der Strom durch die Primärspule 20 durch Trennen aller Einzelvorwiderständen 28 unterbrochen, baut sich das Magnetfeld in der Sekundärspule 18 schlagartig ab und bringt den Schwingkreis aus Sekundärspule 18 und Ausgabekondensator 14 zum Schwingen. Die so erregte Spannung im Ausgabekondensator 14 erzeugt den Therapiepuls 4.

Grundsätzlich kann der Ausgabekondensator 14 beliebig ausgeführt sein, wie beispielsweise in Form von Stichleitungen. In der vorliegenden Ausführung besitzt der Ausgabekondensator 14 eine erste Kondensatorelektrode 34, die an die Sekundärspule 18 angeschlossen ist. Die erste Kondensatorelektrode 34 ist ringförmig ausgebildet und zur Erfassung der Pulsspannung 12 auch mit der Steuervorrichtung 32 verbunden. Der Ausgabekondensator 14 besitzt ferner eine zweite ringförmig ausgebildete Kondensatorelektrode 36, die konzentrisch innerhalb der ersten Kondensatorelektrode 34 angeordnet ist. Die zweite Kondensatorelektrode 36 ist mit Masse 26 verbunden, so dass der an den Transformator 16 über die Sekundärspule 18 angeschlossene Sekundärkreis geschlossen ist. Optional oder zusätzlich kann durch die Umpolung der Ausgangselektroden, hier Kondensatorelektroden genannt, ein inverses Ausrichten des elektrischen Feldes erfolgen, was einen invertierten Therapieimpuls zur Folge hätte. Dann liegt die Ausgangselektrode 34 an Masse und die Ausgangselektrode 36 ist mit der Sekundärspule 18 verbunden.

Zur Verwendung des Elektrotherapiegerätes 2 wird der Ausgabekondensator 14 auf die Haut 6 des Patienten 8 gelegt und durch Ansteuern einer oder mehrerer Einzelvorwiderständen 28 die Sekundärspule 18 über die Primärspule 20 geladen. Durch Trennen aller Einzelvorwiderständen 28 von der Primärspule 20 wird der Therapieimpuls 4 in die Haut 6 des Patienten 8 in der bereits erläuterten Weise eingetragen. Die gesamte Steuerung dieses Vorgangs kann über die Steuervorrichtung 32 erfolgen.

In einer bevorzugten Alternative werden die Einzelwiderstände 28 zur Einstellung des Ladestromes für die Primärspule 18 verwendet. Auf der gemeinsamen Leitung zur Primärspule kann ein weiterer Schalter 48 vorgesehen sein der zwei Funktionen hat.

Eine erste Funktion besteht in der Trennung der Steuerelektronik 32 von der hohen Pulsspannung der Primär- 20 und Sekundärspule 18. Da die Widerstände 28 sehr niederohmig sind würde es zur sofortigen Zerstörung der Steuerelektronik führen, es sei denn, man gestaltet die gesamte Steuerelektronik für den Hochvoltbereich (> 60Vdc).

Eine zweite Funktion besteht in der zentralen zeitlichen Pulsteuerung. Durch die Verwendung dieses zusätzlichen Schalters ist eine einfache und immer zeitlich gleichbleibende Pulsteuerung möglich, unabhängig von der Stellung der Schalter 18 für den Strom zur Primärspule 20.

Um die Therapie zu dokumentieren oder protokollieren, kann die am Ausgabekondensator 14 anliegende Pulsspannung 12 mit der Steuervorrichtung 32 erfasst werden. Aus der Pulsspannung 12 lassen sich Messwerte ableiten, die einen Zustand der Haut beschreiben. Sie kann aus der Pulsspannung 12 beispielsweise die Resonanzfrequenz und darüber die Kapazität der Haut 6 bestimmt werden, die wiederum davon abhängt, wie feucht oder trocken die Haut 6 ist.

Eine Wirkung der Therapie hängt unter anderem davon ab, mit welcher Energie die Pulsspannung 12 in die Haut 6 eingetragen wird. Das zuvor genannte Therapieprotokoll soll helfen, Erfolg der Therapie zu erkennen und die Haut 6 nicht unnötig mit der Pulsspannung 12 zu beaufschlagen.

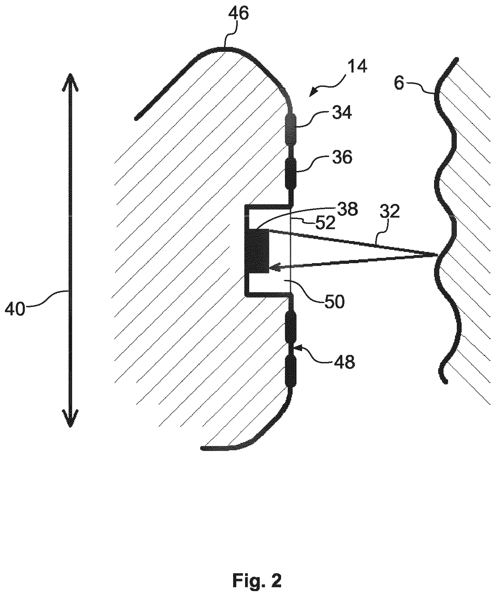

Zur Erstellung eines möglichst präzisen Therapieprotokolls besitzt das Elektrotherapiegerät 2 einen Positionssensor 38 zum Erfassen einer in Fig. 2 angedeuteten Position 40 des Ausgabekondensators 18 auf der Haut 6 während der Abgabe der Pulsspannung 12.

Die erfasste Position 40 kann zusammen mit dem/den die Haut 6 an der erfassten Position 40 beschreibenden Messwert/en in der Steuervorrichtung 32 zu einem Datenpaket 42 zusammengefasst und über eine Schnittstelle 44 als Sendesignal 46 versendet werden. Die Schnittstelle 44 ist in Abhängigkeit der zu verwendenden Versendetechnik, wie Ethernet, Wireless Local Area Network, Nearfield Communication oder Infrarot zu wählen. Als besonders platz-, energie- und bandbreiteschonende Versendetechnik eignet sich die Übertragung per Bluetooth Low Energy.

Es wird auf Fig. 2 Bezug genommen, die eine schematische Darstellung eines Behandlungskopfes 46 des Elektrotherapiegerätes 2 der Fig. 1 zeigt, auf dem erfindungsgemäß der Ausgabekondensator 18 und der Positionssensor 38 angeordnet sind.

Der Behandlungskopf 46 besitzt eine Auflagefläche 48, auf der der Ausgabekondensator 18 angeordnet ist. Die Auflagefläche 48 lässt sich parallel zur Haut 6 ausgerichtet auf diese aufsetzen, so dass der Ausgabekondensator 14 zwischen Behandlungskopf 46 und Haut 6 angeordnet ist.

Innerhalb der zweiten ringförmig ausgebildeten Kondensatorelektrode 36 ist - in Fig. 2 konzentrisch dazu - der Positionssensor 38 in einer Aussparung 50, oder auch Kavität oder Ausnehmung genannt, angeordnet. Die Aussparung 50 ist mit einem transparenten Deckel 52 verschlossen.

Durch den transparenten Deckel 52 beleuchtet der Positionssensor 38 die Haut 6 mit einem einfarbigen oder mehrfarbigen Licht 32. Die Haut 6 reflektiert das Licht 32 und wirft es zum Teil zum Positionssensor 38 zurück. Der Positionssensor 38 nimmt das reflektierte Licht 32 mit einer Photosensorenmatrix auf und wertet das so entstehende Bild aus. Je nachdem ob und wie sich das so entstehende Bild verändert, lässt sich daraus eine Veränderung der Position 40 auf der Haut 6 mit dem Positionssensor 38 erfassen. Auf diese Weise lässt sich die Position 40 des Behandlungskopfes 46 und damit der Ausgabekondensators 14 auf der Haut 6 inkrementell erfassen. Dieses Messprinzip der Position 40 ist aus der Computermaus bestens bekannt, weshalb hierauf nicht weiter eingegangen werden braucht.

Abschließend soll Anhand der Figs. 3 und 4 noch auf die Erstellung beispielhafter Therapieprotokolle eingegangen werden.

In Fig. 3a und 3b ist Kopf des Patienten 8 gezeigt. Er soll der Einfachheit halber als rotationssymmetrisch um die Körperlängsachse angesehen werden.

Auf diese Weise lässt sich der Patient 8 in einem zylindrischen Koordinatensystem mit einer in Richtung der Körperlängsachse verlaufenden Höhenrichtung 54 und einer tangential um die Körperlängsachse verlaufenden Winkelrichtung 56 betrachten. Der Abstand der Hautoberfläche des Patienten 8 von der Körperlängsachse soll der Einfachheit halber als konstant angenommen werden.

Diese Annahmen sind gerechtfertigt, weil der zuvor beschriebene fotosensorisch wirkende Positionssensor 38 die Bewegung des Ausgabekondensators 40 auf der Haut 6 ohnehin nur zweidimensional erfasst und eine radiale Bewegung des Ausgabekondensators 40 ausblendet.

Auf der Haut 6 im Bereich des Kopfes des Patienten befinden sich sechs Therapiepunkte 58, die Ausgabekondensator 40 mit dem Therapiepuls 4 in einer vorbestimmten Stärke beaufschlagen soll.

Der Therapeut bewegt den Behandlungskopf 46 ohne abzusetzen über den Kopf des Patienten 8 und schaltet den Therapiepuls nur an den einzelnen Therapiepunkten 58 ein. Auf diese Weise lässt sich einerseits die Stärke des eingetragenen Therapiepulses 4, aber auch der oben genannte Messwert beispielsweise in Form der Resonanzfrequenz erfassen und im Sendesignal 46 zur weiteren Auswertung versenden.

Dabei ist die Positionsbestimmung am Rücken eines Patienten mit einer zweidimensionalen Positionsbestimmung exakter als im dreidimensionalen Raum, beispielsweise am Kopf. Bei dieser Art des Protokolls läuft das Verfahren so, dass die zeitliche Abfolge der Hautkontakte die Zuordnung zu den Therapiepunkten (58a - 58f) herstellt.

Beispiel: Die Anwendung beginnt mit dem Punkt 58a. Der Behandlungskopf 40 wird auf dem Punkt 58a abgesetzt, die Steuerelektronik erkennt optisch den Hautkontakt, beginnt mit dem Therapieimpuls und misst über die Veränderung der Parameter der Resonanzfrequenz die Haut- und Gewebereaktion (Bio-Feed-Back). Nach einem definierten Ereignis (zeitlich oder auf Grund bestimmter Resonanzparameter) erfolgt ein automatischer Eintrag in digitale Therapieprotokoll via Sendesignal 46. Der Anwender wird aufgefordert den Behandlungskopf 40 von der Haut zu lösen und zum nächsten Therapiepunkt z.B. 58b zu gehen. Die Prozedur wiederholt sich mit der Anzahl der Therapiepunkte (hier im Gesicht 6) und mit der Anzahl der durch zu führenden Therapiedurchläufen.

Es ist allerdings möglich die Positionsbestimmung, um eine dritte Dimension zu erweitern oder die Geräteausrichtung zu bestimmen, welche bei der Beurteilung der Genauigkeit berücksichtigt wird. Dies kann einen geeigneten Sensor, wie z.B. durch ein Gyroskop, erreicht werden. Derartige Sensoren finden bereits in der Mobiltelefonanwendung umfangreich Anwendung

In gleicher Weise lässt sich dies auch am in den Figs. 4a und 4b gezeigten Rücken des Patienten 8 umsetzen.

Wie am Kopf sind auch am Rücken sechs Therapiepunkte 58 hier im Bereich des Nackens angeordnet, die sich auch in der gleichen Weise behandeln lassen. Die Anwendung für diese Therapiepunkte erfolgt wir zuvor erläutert.

Zusätzlich verlaufen auf dem Rücken noch drei Therapiebahnen 60, über die der Therapeut den Behandlungskopf 46 bewegen kann, ohne den Therapiepuls 4 abzuschalten. Die Daten auf diesen Therapiebahnen 60 lassen sich in Abhängigkeit der örtlichen Auflösungsgenauigkeit des Positionssensors 38 in bestimmten örtlichen Abtastabständen kontinuierlich erfassen und in dem Sendesignal 46 versenden. Insbesondere kann die Positionsfeststellung in der Ebene wie folgt erfolgen: Über die dx und dy - Veränderungen lässt sich beginnend von einem vorgegebenen Therapiestartpunkt (Erkennung Hautkontakt) der Weg des Behandlungskopfes 46 nachverfolgen, nach definierten Abständen via Sendesignal 46 ein automatischer Eintrag ins digitale Therapieprotokoll initiieren oder dem Therapeuten durch Ton- oder Lichtsignal auf eine Abweichung von der Therapiebahn signalisieren (Überwachungsfunktion). Bitte beachten das eine Therapie nach einem Muster bzw. nach Regeln erfolgt, die digital (Grafikdisplay auf einem Tablet oder PC) vorgegeben, nachvollzogen und überwacht werden.

Optional oder zusätzlich kann ein G-Sensor integriert, der die Beschleunigungen in alle 3 Achsen ermittelt. Die Informationen dieses Sensors werden zur Unterstützung und Validierung der Bewegung des optischen Wegsensors verwendet. Die alleinige Verwendung der Informationen eines G-Sensors reichen nicht aus, um einen Weg zu beschreiben, da bei einer gleichmäßigen Geschwindigkeit (Beschleunigung = 0) keine Bewegungsinformationen vorliegen. Es lässt sich aber sehr gut der Beginn und das Ende sowie die Richtung einer Bewegung feststellen, auch im Raum also auf der X-, Y- und Z-Achse. Die Ermittlung des Weges von A nach B erfolgt wie beschrieben durch den dx/dy-Wegsensor.

Es ist auch somit möglich die Relativbewegung von einem Therapiepunkt zum nächsten ohne Hautkontakt zumindest zu interpolieren.

Weiterhin kann das erfindungsgemäße Elektrotherapiegerät einen oder mehrere Abstandssensoren, z.B. Lasersensoren aufweisen, welches Hautunebenheiten misst und diese bei der Signalverarbeitung berücksichtigt.

Weiterhin kann das erfindungsgemäße Elektrotherapiegerät einen oder mehrere Kraft- bzw. Drucksensoren enthalten, um die Kraft des Aufdruckes des Behandlungskopfes auf die Hautoberfläche zu messen und um den Therapeuten durch Licht und/oder akustische Signale zu einer gleichmäßigen und effektiven taktilen Behandlung zu führen.

## Patentansprüche

1. Elektrotherapiegerät (2) zum Eintragen eines elektrischen Therapieimpulses (4) in die Haut (6) eines Patienten (8), umfassend:
- eine Pulsquelle (10) zur Ausgabe einer Pulsspannung (12);
- einen auf die Haut (6) des Patienten (8) auflegbaren Ausgabekondensator (14), der mit einer ersten an einen ersten Pol der Pulsquelle (10) angeschlossen Kondensatorelektrode (34) und mit einer zweiten an einen zweiten Pol der Pulsquelle (10) angeschlossen Kondensatorelektrode (36) eingerichtet ist, die Pulsspannung (12) in den Therapiepuls (4) zu wandeln;
**gekennzeichnet durch**
einen Positionssensor (38) zum Erfassen einer Position (40) des Ausgabekondensators (14) auf der Haut (6) während der Abgabe des Therapiepulses (4).

2. Elektrotherapiegerät (2), nach Anspruch 1, **dadurch gekennzeichnet, dass** die Pulsquelle (10) einen Schwingkreis mit dem Ausgabekondensator (14) und einer magnetisch ladbaren Spule (18) umfasst, die vorzugsweise Teil eines Transformators (16) ist.

3. Elektrotherapiegerät (2) nach Anspruch 1 oder 2, **dadurch gekennzeichnet, dass** umfassend eine Sendeschnittstelle (44) zur Übertragung der Position (40) des Ausgabekondensators (14) an eine
Datenverarbeitungseinrichtung.

4. Elektrotherapiegerät (2) nach Anspruch 3, **dadurch gekennzeichnet, dass** die Sendeschnittstelle (44) eingerichtet ist, die Position (40) des Ausgabekondensators (14) gemeinsam mit einem die Haut (6) an der erfassten Position (40) beschreibenden Messwert (12) zu versenden.

5. Elektrotherapiegerät (2) nach einem der vorstehenden Ansprüche, **dadurch gekennzeichnet, dass** der Positionssensor (38) eingerichtet ist, die Position des Ausgabekondensators (14) auf der Haut (6) inkrementell zu erfassen.

6. Elektrotherapiegerät (2) nach Anspruch 5, **dadurch gekennzeichnet, dass** der Positionssensor (38) zur inkrementellen Positionserfassung eingerichtet ist, in regelmäßigen Abständen ein Bild (32) der Haut (6) zu erfassen und mit einem zuvor aufgenommenen Bild (32) der Haut (6) zu vergleichen.

7. Elektrotherapiegerät (2) nach einem der vorstehenden Ansprüche, **dadurch gekennzeichnet, dass** die Kondensatorelektroden (34, 36) als konzentrisch zueinander angeordnete geschlossene Schleifen ausgebildet sind.

8. Elektrotherapiegerät (2) nach Anspruch 7, **dadurch gekennzeichnet, dass** der Positionssensor (38) innerhalb der inneren Schleife (36) der konzentrisch zueinander angeordneten Schleifen (36) angeordnet ist.

9. Elektrotherapiegerät (2) nach einem der vorstehenden Ansprüche, **dadurch gekennzeichnet, dass** der Abgabekondensator (14) an einem Behandlungskopf (46) mit einer den Positionssensor (38) aufnehmenden Aussparung (50) gehalten ist.

10. Elektrotherapiegerät (2) nach einem der vorhergehenden Ansprüche, **dadurch gekennzeichnet, dass** die Aussparung (50) mit einer Abdeckung (52) verschlossen ist, die bevorzugt transparent ist.

11. Elektrotherapiegerät (2) nach einem der vorstehenden Ansprüche, **dadurch gekennzeichnet, dass** das Elektrotherapiegerät zudem einen Beschleunigungssensor zur Beurteilung der Positionserfassung des Positionssensors (38) aufweist.

## Claims

1. Electrotherapy device (2) for applying an electrical therapy pulse (4) to the skin (6) of a patient (8), comprising:
- a pulse source (10) for outputting a pulse voltage (12);
- an output capacitor (14) which can be placed on the skin (6) of the patient (8), comprises a first capacitor electrode (34) connected to a first terminal of the pulse source (10) and a second capacitor electrode (36) connected to a second terminal of the pulse source (10) and is adapted to convert the pulse voltage (12) into the therapy pulse (4);
**characterized by**
a position sensor (38) for sensing a position (40) of the output capacitor (14) on the skin (6) during delivery of the therapy pulse (4).

2. Electrotherapy device (2) according to claim 1, **characterized in that** the pulse source (10) comprises an oscillating circuit with the output capacitor (14) and a magnetically chargeable coil (18), which is preferably part of a transformer (16).

3. Electrotherapy device (2) according to claim 1 or 2, **characterized in that** comprising a transmitting interface (44) for transmitting the position (40) of the output capacitor (14) to a data processing device.

4. Electrotherapy device (2) according to claim 3, **characterized in that** the transmitting interface (44) is adapted to transmit the position (40) of the output capacitor (14) together with a measured value (12) describing the skin (6) at the detected position (40).

5. Electrotherapy device (2) according to one of the preceding claims, **characterized in that** the position sensor (38) is adapted to incrementally detect the position of the output capacitor (14) on the skin (6).

6. Electrotherapy device (2) according to claim 5, **characterized in that** the position sensor (38) for incremental position detection is adapted to capture an image (32) of the skin (6) at regular intervals and to compare it with a previously captured image (32) of the skin (6).

7. Electrotherapy device (2) according to one of the preceding claims, **characterized in that** the capacitor electrodes (34, 36) are designed as closed loops arranged concentrically to one another.

8. Electrotherapy device (2) according to claim 7, **characterized in that** the position sensor (38) is arranged within the inner loop (36) of the loops (36) arranged concentrically to each other.

9. Electrotherapy device (2) according to one of the preceding claims, **characterized in that** the output capacitor (14) is held on a treatment head (46) having a recess (50) receiving the position sensor (38).

10. Electrotherapy device (2) according to one of the preceding claims, **characterized in that** the recess (50) is closed with a cover (52) which is preferably transparent.

11. Electrotherapy device (2) according to one of the preceding claims, **characterized in that** the electrotherapy device further comprises an acceleration sensor for assessing the position detection of the position sensor (38).

## Revendications

1. Appareil d'électrothérapie (2) pour appliquer une impulsion électrique de traitement (4) dans la peau (6) d'un patient (8), comprenant :
- une source d'impulsions (10) destinée à émettre une tension en impulsions (12) ;
- un condensateur de sortie (14) pouvant être posé sur la peau (6) du patient (8), qui est équipé d'une première électrode de condensateur (34) pouvant être raccordée à un premier pôle de la source d'impulsions (10) et d'une deuxième électrode de condensateur (36) raccordée à un deuxième pôle de la source d'impulsions (10) pour convertir la tension en impulsions (12) en impulsion de traitement (4) ;
**caractérisé en ce qu'**il comprend un capteur de position (38) destiné à détecter une position (40) du condensateur de sortie (14) sur la peau (6) pendant l'émission de l'impulsion de traitement (4).

2. Appareil d'électrothérapie (2) selon la revendication 1, **caractérisé en ce que** la source d'impulsions (10) comprend un circuit oscillant avec le condensateur de sortie (14) et une bobine (18) pouvant être chargée magnétiquement, qui fait de préférence partie d'un transformateur (16).

3. Appareil d'électrothérapie (2) selon la revendication 1 ou 2, **caractérisé en ce qu'**il comprend une interface d'émission (44) pour transmettre la position (40) du condensateur de sortie (14) à un dispositif de traitement des données.

4. Appareil d'électrothérapie (2) selon la revendication 3, **caractérisé en ce que** l'interface d'émission (44) est conçue pour émettre la position (40) du condensateur de sortie (14) en même temps qu'une valeur de mesure (12) décrivant la peau (6) dans la position (40) détectée.

5. Appareil d'électrothérapie (2) selon l'une des revendications précédentes, **caractérisé en ce que** le capteur de position (38) est conçu pour détecter la position du condensateur de sortie (14) sur la peau (6) de façon incrémentielle.

6. Appareil d'électrothérapie (2) selon la revendication 5, **caractérisé en ce que** le capteur de position (38) pour la détection incrémentielle de la position est conçu pour acquérir à intervalles réguliers une image (32) de la peau (6) et la comparer avec une image (32) de la peau (6) acquise précédemment.

7. Appareil d'électrothérapie (2) selon l'une des revendications précédentes, **caractérisé en ce que** les électrodes de condensateur (34, 36) sont conformées comme des boucles fermées disposées de façon concentrique l'une de l'autre.

8. Appareil d'électrothérapie (2) selon la revendication 7, **caractérisé en ce que** le capteur de position (38) est disposé à l'intérieur de la boucle intérieure (36) des boucles (36) disposées de façon concentrique l'une de l'autre.

9. Appareil d'électrothérapie (2) selon l'une des revendications précédentes, **caractérisé en ce que** le condensateur d'émission (14) est retenu sur une tête de traitement (46) avec un creux (50) recevant le capteur de position (38).

10. Appareil d'électrothérapie (2) selon l'une des revendications précédentes, **caractérisé en ce que** le creux (50) est fermé par un couvercle (52) qui est de préférence transparent.

11. Appareil d'électrothérapie (2) selon l'une des revendications précédentes, **caractérisé en ce que** l'appareil d'électrothérapie présente en outre un capteur d'accélération pour évaluer la détection de la position par le capteur de position (38).
